# EUROPEAN PATENT APPLICATION

(11) **EP 3 960 751 A1**
(43) Date of publication of application: **02.03.2022**
(21) Application number: 20794466.1
(22) Date of filing: 23.04.2020
(51) Int. Cl.: C07K 1/02, C07K 1/06

(54) **METHOD FOR PRODUCING PEPTIDE COMPOUND**

(30) Priority: 25.04.2019 JP 2019084005
(71) Applicant: Nissan Chemical Corporation, Tokyo 103-6119 (JP)
(72) Inventor: HANDA Michiharu, Funabashi-shi, Chiba 274-8507 (JP); MURASE Shota, Funabashi-shi, Chiba 274-8507 (JP)
(74) Representative: dompatent von Kreisler Selting Werner - Partnerschaft von Patent- und Rechtsanwälten mbB
(86) International application number: PCT/JP2020/017477
(87) International publication number: WO 2020/218412

(57) **Abstract**

An object of the present invention is to provide a method for producing a peptide with high efficiency.

Provided is a method for producing a peptide which comprises the following step (1):

(1) a step of reacting a peptide in which a C-terminal carboxy group is activated which is obtained by reacting an N-protected peptide represented by the formula (I): 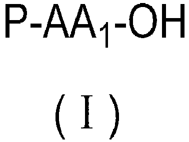 a tertiary amine represented by the formula (II): 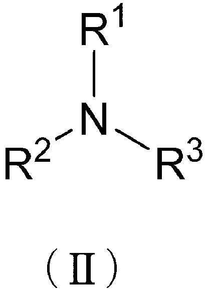 and an acid halide in a flow reactor,
with a silylated amino acid or peptide obtained by reacting an amino acid or peptide represented by the formula (III): 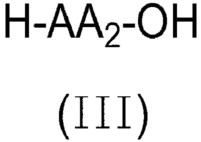 with a silylating agent,
in a flow reactor
[in the formulae (I) to (III), AA₁, AA₂, P, R¹, R² and R³ are as defined in the present specification and the claims].

## Description

### TECHNICAL FIELD

The present invention relates to a novel method for producing a peptide using a flow reactor.

### BACKGROUND ART

It has been known a method in which a C-terminal carboxy group in a peptide is activated by an alkyl chloroformate and reacted with a silylated amino acid or peptide to synthesize a peptide in which the carboxylic acid is liberated with one step (Patent Documents 1 and 2). Incidentally, it has been known that, in the method in which a C-terminal carboxy group in an amino acid an N-terminal of which has been protected by an alkoxycarbonyl group is activated using an alkyl chloroformate, an activated intermediate can be stably taken out (Non-Patent Documents 1 and 2). On the other hand, it has been known that, for example, in the method of activating a C-terminal carboxy group in an amino acid having an amide structure in an N-terminal such as a peptide using an alkyl chloroformate, an activated intermediate cannot be taken out stably and formation of azlactone (oxazolone) proceeds (Non-Patent Document 1).

Also, there is a continuous system as a reaction system different from a batch system, and among these, it has been known a method in which chemical synthesis is carried out continuously while flowing a solution using a small type reaction apparatus called a flow reactor and a microreactor. It has been known that the flow reactor has the advantages that it is possible to carry out precise temperature control and reaction time control, and has good mixing efficiency since the reaction is carried out using a small reaction vessel as compared with the batch system which has been conventionally carried out (Non-Patent Document 5). As an example of synthesizing a peptide, it has been known a method in which a dipeptide is synthesized using triphosgene as a condensation agent and elongating the peptide chain toward the N-terminal direction (Non-Patent Documents 3 and 4). Also, it has been known a method in which peptides are condensed with each other using O-(benzotriazol-1-yl)-N,N,N',N'-tetramethyl-uronium hexafluorophosphate (HBTU) as a condensation agent (Patent Documents 3 and 4).

### PRIOR ART DOCUMENTS

### PATENT DOCUMENTS

Patent Document 1: JP Patent No. 5,535,928
Patent Document 2: US Patent No. 5,714,484
Patent Document 3: JP 2006-169165A
Patent Document 4: WO 2007/059922

### NON-PATENT DOCUMENTS

Non-Patent Document 1: International Journal of Peptide and Protein Research, 1988, vol. 31, pp. 339-344
Non-Patent Document 2: Canadian Journal of Chemistry, 1987, vol. 67, pp. 619-624
Non-Patent Document 3: Angewandte Chemie International Edition, 2014, vol. 53, pp. 851-855
Non-Patent Document 4: Nature Communications, 2016, vol. 7, 13429
Non-Patent Document 5: Development and application of microreactor, 2003, pp. 3-9

### SUMMARY OF THE INVENTION

### PROBLEMS TO BE SOLVED BY THE INVENTION

When the present inventors have confirmed, in the batch system described in Patent Documents 1 and 2, it was found that in order to control heat generation in the reaction of the peptide in which the carboxy group has been activated and the silylated amino acid or peptide, it takes a long time to drop a reagent, whereby azlactone, which is a by-product, is generated and the yield is lowered, so that it is not suitable for industrial implementation.

On the other hand, in the flow system described in Non-Patent Documents 3 and 4, an amino acid in which a C-terminal is protected by an alkyl group is used, so that when the peptide is to be elongated toward the C-terminal direction, deprotection of the C-terminal is to be required. Also, activation of the carboxy group is limited only to the amino acid in which the amino group is protected, and no investigation on the elongation to the C-terminal side by activation of the carboxy group at the peptide terminal having an amide bond, which easily forms azlactone, has been carried out.

Further, in the flow system described in Patent Documents 3 and 4, the C-terminal of the obtained peptide is an alkylamide that is difficult to deprotect, and is not suitable for the method of elongating the peptide chain to the C-terminal side. And when the reaction of the peptide in which the C-terminal carboxy group is activated by HBTU and the silylated amino acid or peptide is carried out, it was found that it generated clogging of the flow path due to precipitation of a solid.

Moreover, in the flow system, as an organic tertiary amine used at the time of activating a carboxy group with an alkyl chloroformate, when triethylamine or diisopropylethylamine which has been used in the batch system is used, it was found that the reaction does not proceed sufficiently.

The present invention is to provide a novel method for producing a peptide which comprises elongating a peptide chain from a C-terminal side using a flow reactor.

### MEANS TO SOLVE THE PROBLEMS

The present inventors have earnestly studied and as a result, they have found that the above-mentioned problems can be solved by subjecting to the reaction of a peptide in which a C-terminal carboxy group of the peptide is activated using an acid halide and an organic tertiary amine having a specific structure in a flow reactor so that the C-terminal carboxy group has been activated, and a silylated amino acid or peptide in a flow reactor, whereby they have accomplished the present invention. That is, the present invention has the following characteristics.

[1] A method for producing a peptide which comprises the following step (1):
   (1) a step of reacting a peptide in which a C-terminal carboxy group is activated which is obtained by reacting an N-protected peptide represented by the formula (I): 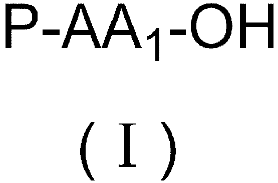
      [wherein AA₁ represents a group derived from a peptide comprising a 2 to 20 amino acids, and P represents an N-terminal protective group],
      a tertiary amine represented by the formula (II): 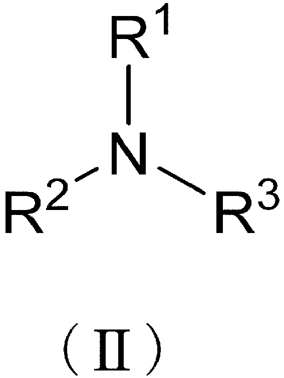
      [wherein
         among R¹, R² and R³, one or two is/are a methyl group(s), the remainder(s) is/are an aliphatic hydrocarbon group(s) which may have a substituent(s), when one of R¹, R² and R³ is a methyl group, the remaining two may form a 6 to 7-membered ring, together with the nitrogen atom to which they are bonded, by combining them to form a C₅₋₆ alkylene chain and, in this case, one of the alkylene chains may be replaced with O or NR⁸ (R⁸ represents an aliphatic hydrocarbon group which may have a substituent(s))] and an acid halide in a flow reactor,
      with a silylated amino acid or peptide obtained by reacting an amino acid or peptide represented by the formula (III): 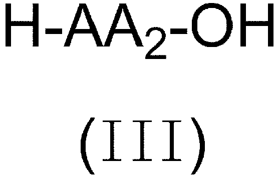
      [wherein AA₂ represents an amino acid or a group derived from a peptide having 2 to 20 residues] with a silylating agent,
         in a flow reactor.
[2] The method for producing a peptide described in [1], wherein an amount of the tertiary amine represented by the formula (II) to be used is 0.05 mol equivalent to 1 mol equivalent based on the N-protected peptide represented by the formula (I), and further using a tertiary amine represented by the formula (IV): 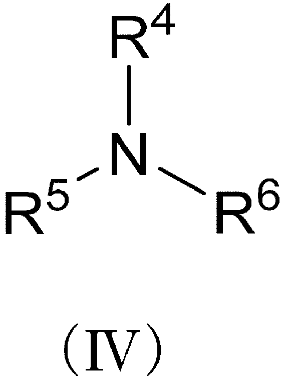 [wherein
   R⁴, R⁵ and R⁶ each independently represent an aliphatic hydrocarbon group (provided that a methyl group is excluded) which may have a substituent(s), two of R⁴, R⁵ and R⁶ may form a 6 to 7-membered ring, together with the nitrogen atom to which they are bonded, by combining them to form a C₅₋₆ alkylene chain and, in this case, one of the alkylene chains may be replaced with O or NR⁸ (R⁸ represents an aliphatic hydrocarbon group which may have a substituent(s))].
[3] The method for producing a peptide described in [2], wherein a combined amount of the tertiary amine represented by the formula (IV) and the tertiary amine represented by the formula (II) to be used is 1.0 to 10 mol equivalents based on the N-protected peptide represented by the formula (I).
[4] The method for producing a peptide described in any one of [1] to [3], wherein the acid halide is an alkyl chloroformate, carboxylic acid chloride, sulfonyl chloride or phosphoryl chloride.
[5] The method for producing a peptide described in any one of [1] to [4], wherein the acid halide is a C₁₋₆ alkyl chloroformate.
[6] The method for producing a peptide described in any one of [1] to [5], wherein the acid halide is isobutyl chloroformate.
[7] The method for producing a peptide described in any one of [1] to [6], wherein the tertiary amine represented by the formula (II) is N,N-dimethylbutylamine, N,N-dimethylbenzylamine, N-methyldiethylamine, N-methylpiperidine or N-methylmorpholine.
[8] The method for producing a peptide described in any one of [2] to [7], wherein the tertiary amine represented by the formula (IV) is N,N-diisopropylethylamine or tri-n-propylamine.
[9] The method for producing a peptide described in any one of [1] to [8], wherein the amino acid or peptide is constituted by an α-amino acid.
[10] The method for producing a peptide described in any one of [1] to [9], wherein the protective group of the N-terminal of the N-protected peptide is a carbamate-based protective group.
[11] The method for producing a peptide described in any one of [1] to [10], wherein a protective group of the N-terminal of the N-protected peptide is a benzyloxycarbonyl group, a 9-fluorenylmethoxycarbonyl group or a t-butoxycarbonyl group.
[12] The method for producing a peptide described in any one of [1] to [11], wherein the silylating agent is N,O-bis(trimethylsilyl)acetamide.

### EFFECTS OF THE INVENTION

According to the present invention, a novel method for producing a peptide by a flow reactor could be provided.

### EMBODIMENTS TO CARRY OUT THE INVENTION

In the present specification, "n-" means normal, "i" means iso, "s-" and "sec" means secondary, "t-" and "tert-" means tertiary, "c-" means cyclo, "p-" means para, "Me" means methyl, "Bu" means butyl, "Pr" means propyl, "Bn" means benzyl, "Ph" means phenyl, "Boc" means t-butoxycarbonyl, "Cbz" means benzyloxycarbonyl, "Fmoc" means 9-fluorenylmethoxycarbonyl, "Trt" means trityl and "Ac" means acetyl.

The term "halogen" means fluorine, chlorine, bromine or iodine.

The terms "C₁₋₆ alkyl group" mean a linear or branched alkyl group having 1 to 6 carbon atoms, and specific examples thereof may be mentioned a methyl group, an ethyl group, an n-propyl group, an isopropyl group, an n-butyl group, an isobutyl group, an s-butyl group, a t-butyl group, an n-pentyl group, an n-hexyl group, etc.

The terms "aliphatic hydrocarbon group" mean a linear, branched or cyclic, saturated or unsaturated aliphatic hydrocarbon group, there may be mentioned an alkyl group, a cycloalkyl group, an alkenyl group, an alkynyl group, an aralkyl group, etc., and specific examples thereof may be mentioned a C₁₋₁₀ alkyl group, a C₃₋₆ cycloalkyl group, a C₂₋₆ alkenyl group, a C₂₋₆ alkynyl group, a C₇₋₁₄ aralkyl group, etc.

The terms "C₁₋₁₀ alkyl group" mean a linear or branched alkyl group having 1 to 10 carbon atoms, and specific examples thereof may be mentioned a methyl group, an ethyl group, an n-propyl group, an isopropyl group, an n-butyl group, an isobutyl group, an s-butyl group, a t-butyl group, an n-pentyl group, an n-hexyl group, an octyl group, a decyl group, etc.

The terms "C₂₋₆ alkyl group" mean a linear or branched alkyl group having 2 to 6 carbon atoms, and specific examples thereof may be mentioned an ethyl group, an n-propyl group, an isopropyl group, an n-butyl group, an isobutyl group, an s-butyl group, a t-butyl group, an n-pentyl group, an n-hexyl group, etc.

The terms "C₂₋₆ alkenyl group" mean a linear or branched alkenyl group having 2 to 6 carbon atoms, and specific examples thereof may be mentioned a vinyl group, a 1-propenyl group, an allyl group, an isopropenyl group, a butenyl group, an isobutenyl group, etc.

The terms "C₂₋₆ alkynyl group" mean a linear or branched alkynyl group having 2 to 6 carbon atoms, and specific examples thereof may be mentioned an ethynyl group, a 1-propynyl group, etc.

The terms "C₃₋₆ cycloalkyl group" mean a cycloalkyl group having 3 to 6 carbon atoms, and specific examples thereof may be mentioned a cyclopropyl group, a cyclobutyl group, a cyclopentyl group, a cyclohexyl group, etc.

The terms "C₇₋₁₄ aralkyl group" mean an aralkyl group having 7 to 14 carbon atoms, and specific examples thereof may be mentioned a benzyl group, a 1-phenylethyl group, a 2-phenylethyl group, a 1-phenylpropyl group, a naphthylmethyl group, a 1-naphthylethyl group, a 1-naphthylpropyl group, etc.

The terms "tri-C₁₋₆ alkylsilyl group" mean a group in which the same or different three above-mentioned "C₁₋₆ alkyl groups" are bonded to the silyl group, and specific examples thereof may be mentioned a trimethylsilyl group, a triethylsilyl group, a triisopropylsilyl group, a t-butyldimethylsilyl group, etc.

The terms "tri-C₁₋₆ alkylsilyloxy group" mean a group in which the same or different three above-mentioned "C₁₋₆ alkyl groups" are bonded to the silyloxy group, and specific examples thereof may be mentioned a trimethylsilyloxy group, a triethylsilyloxy group, a triisopropylsilyloxy group, a t-butyldimethylsilyloxy group, etc.

The terms "di-C₁₋₆ alkylamino group" mean the same or different two above-mentioned "C₁₋₆ alkyl groups" are bonded to the amino group, and specific examples thereof may be mentioned a dimethylamino group, a diethylamino group, a di-n-propylamino group, a diisopropylamino group, a di-n-butylamino group, a diisobutyl-amino group, a di-t-butylamino group, a di-n-pentylamino group, a di-n-hexylamino group, an N-ethyl-N-methylamino group, an N-methyl-N-n-propylamino group, an N-isopropyl-N-methylamino group, an N-n-butyl-N-methylamino group, an N-isobutyl-N-methylamino group, an N-t-butyl-N-methylamino group, an N-methyl-N-n-pentylamino group, an N-n-hexyl-N-methylamino group, an N-ethyl-N-n-propylamino group, an N-ethyl-N-isopropylamino group, an N-n-butyl-N-ethylamino group, an N-ethyl-N-isobutylamino group, an N-t-butyl-N-ethylamino group, an N-ethyl-N-n-pentylamino group, an N-ethyl-N-n-hexylamino group, etc.

The terms "5 to 10-membered heterocyclic group" mean a monocyclic-based or a fused ring-based heterocyclic group having a number of the atoms constituting the ring of 5 to 10, and containing 1 to 4 hetero atoms independently selected from the group consisting of a nitrogen atom, an oxygen atom and a sulfur atom in the atoms constituting the ring. The heterocyclic group may be either of saturated, partially unsaturated or unsaturated, and specific examples thereof may be mentioned a pyrrolidinyl group, a tetrahydrofuryl group, a tetrahydrothienyl group, a piperidyl group, a tetrahydropyranyl group, a tetrahydrothiopyranyl group, a pyrrolyl group, a furyl group, a thienyl group, a pyridyl group, a pyrimidinyl group, a pyridazinyl group, an azepanyl group, an oxepanyl group, a thiepanyl group, an azepinyl group, an oxepinyl group, a thiepinyl group, an imidazolyl group, a pyrazolyl group, an oxazolyl group, a thiazolyl group, an imidazolinyl group, a pyrazinyl group, a morpholinyl group, a thiazinyl group, an indolyl group, an isoindolyl group, a benzimidazolyl group, a purinyl group, a quinolyl group, an isoquinolyl group, a quinoxalinyl group, a cinnolinyl group, a pteridinyl group, a chromenyl group, an isochromenyl group, etc.

The terms "C₆₋₁₄ aryl group" mean an aromatic hydrocarbon group having 6 to 14 carbon atoms, and specific examples thereof may be mentioned a phenyl group, a 1-naphthyl group, a 2-naphthyl group, a 1-anthracenyl group, a 2-anthracenyl group, a 9-anthracenyl group, a biphenyl group, etc.

The terms "C₆₋₁₄ aryloxy group" mean an aryloxy group having 6 to 14 carbon atoms, and specific examples thereof may be mentioned a phenoxy group, a 1-naphthyloxy group, a 2-naphthyloxy group, a 1-anthracenyloxy group, a 2-anthracenyloxy group, a 9-anthracenyloxy group, a biphenyloxy group, etc..

The terms "C₁₋₆ alkoxy group" mean a linear or branched alkoxy group having 1 to 6 carbon atoms, and specific examples thereof may be mentioned a methoxy group, an ethoxy group, an n-propoxy group, an isopropoxy group, an n-butoxy group, an isobutoxy group, a t-butoxy group, an n-pentyloxy group, an n-hexyloxy group, etc.

The terms "C₃₋₆ cycloalkoxy group" mean a cycloalkoxy group having 3 to 6 carbon atoms, and specific examples thereof may be mentioned a cyclopropoxy group, a cyclobutoxy group, a cyclopentyloxy group, a cyclohexyloxy group, etc.

The terms "C₁₋₆ alkoxycarbonyl group" mean a linear or branched alkoxycarbonyl group having 1 to 6 carbon atoms, and specific examples thereof may be mentioned a methoxycarbonyl group, an ethoxycarbonyl group, an n-propoxycarbonyl group, an isopropoxycarbonyl group, an n-butoxycarbonyl group, an isobutoxycarbonyl group, a t-butoxycarbonyl group, an n-pentyloxycarbonyl group, an n-hexyloxycarbonyl group, etc.

The terms "which may have a substituent(s)" mean that it is unsubstituted, or substituted by an optional number of an optional substituent(s).

With regard to the above-mentioned "optional substituent(s)", the kind thereof is not particularly limited as long as it is a substituent which does not exert any bad effect to the reaction which is the target of the present invention.

As the "substituent(s) in the "aliphatic hydrocarbon group which may have a substituent(s)", there may be mentioned, for example, a C₆₋₁₄ aryl group, a C₆₋₁₄ aryl oxy group, a 5 to 10-membered heterocyclic ring group, a hydroxy group, a C₁₋₆ alkoxy group, a C₃₋₆ cycloalkoxy group, an acetoxy group, a benzoyloxy group, an amino group, an N-acetylamino group, a di-C₁₋₆ alkylamino group, a halogen atom, a C₁₋₆ alkoxycarbonyl group, a phenoxycarbonyl group, an N-methylcarbamoyl group, an N-phenylcarbamoyl group, a tri-C₁₋₆ alkylsilyl group, a tri-C₁₋₆ alkylsilyloxy group, a cyano group, a nitro group, a carboxyl group, etc., preferably a C₆₋₁₄ aryl group, a C₁₋₆ alkoxy group, a di-C₁₋₆ alkylamino group, a tri-C₁₋₆ alkylsilyl group and a tri-C₁₋₆ alkylsilyloxy group, and more preferably a C₆₋₁₄ aryl group, a C₁₋₆ alkoxy group and a tri-C₁₋₆ alkylsilyl group.

The terms "N-protected peptide" mean a peptide in which the amino group at the N-terminal is protected, and the carboxyl group at the C-terminal is unprotected.

In the present specification, specific examples of the expression "when one of R¹, R² and R³ is a methyl group, the remaining two may form a 6 to 7-membered ring, together with the nitrogen atom to which they are bonded, by combining them to form a C₅₋₆ alkylene chain and, in this case, one of the alkylene chains may be substituted with O or NR⁸ (R⁸ represents an aliphatic hydrocarbon group which may have a substituent(s))" may be mentioned piperidine, azepane, morpholine, N-methylpiperazine, etc.

The amino acid(s) used in the present invention is an organic compound having both functional groups of an amino group and a carboxy group, preferably α-amino acid, β-amino acid, γ-amino acid or δ-amino acid, more preferably α-amino acid or β-amino acid, and further preferably α-amino acid.

The amino group of the amino acid used in the present invention may be substituted, preferably may be substituted by an aliphatic hydrocarbon group which may have a substituent(s), more preferably it may be substituted by a C₁₋₆ alkyl group or a C₇₋₁₄ aralkyl group, and further preferably it may be substituted by a methyl group.

The amino acid(s) constituting the peptide used in the present invention is/are the above-mentioned amino acid(s).

The steric structure of the α-amino acid is not particularly limited, and preferably an L-isomer.

All technical terms and scientific terms used in the present specification have the same meanings as those commonly understood by those skilled in the art to which the present invention belongs. The same or equivalent optional method and material described in the present specification can be used in practice or experiment of the present invention, and preferable methods and materials are described below. All publications and patents referred to in the present specification are incorporated in the present specification by reference, for example, for the purpose of describing and disclosing the constructs and methodologies, which are described in the publications capable of using in connection with the described inventions.

### (Specific explanation of method for producing peptide of present invention)

Hereinafter, the process for producing a peptide of the present invention will be explained.

This specific explanation is explained based on the following.
(a) R¹, R² and R³ have the same meanings as defined above.
(b) The specific conditions of the reaction are not particularly limited as long as production of the peptide of the present invention is accomplished. Preferable conditions in each reaction are appropriately described in detail.
(c) The solvent(s) described in each reaction may be used singly, or may be used in admixture of two or more kinds.

The present invention is directed to a producing method in which a carboxy group at the C-terminal of an N-protected peptide is activated using an organic tertiary amine having a specific structure in a flow reactor, and a silylated amino acid or peptide is reacted therewith in a flow reactor to obtain a peptide in which the C-terminal is elongated.

The activation reaction of the C-terminal carboxy group of the N-protected peptide using an organic tertiary amine having a specific structure can be carried out in the presence of an acid chloride. Also, when it is carried out using an organic tertiary amine having a specific structure as a catalyst, it can be carried out by co-presenting other bases.

In the present specification, the terms "organic tertiary amine having a specific structure" mean the amine compound represented by the above-mentioned formula (II), preferably an amine in which among R¹, R² and R³, one or two is/are a methyl group(s), and the remainder is a C₁₋₆ alkyl group which may have a substituent(s), or an amine in which one of R¹, R² and R³ is a methyl group, and the remaining two are combined together to form a Cs alkylene chain whereby forming a 6-membered ring with the nitrogen atom to which they are bonded, and in this case, one of the alkylene chains may be replaced with O, and more preferably N,N-dimethylbutylamine, N,N-dimethylbenzylamine, N-methyldiethylamine, N-methylpiperidine or N-methylmorpholine.

The acid halide to be used in the present invention is preferably an alkyl chloroformate, carboxylic acid chloride, sulfonyl chloride, phosphoryl chloride, more preferably an alkyl chloroformate, carboxylic acid chloride, and further preferably a C₁₋₆ alkyl chloroformate, and particularly preferably isobutyl chloroformate.

The "other bases" to be used in the present invention are amine compounds represented by the above-mentioned formula (IV), preferably an amine in which R⁴, R⁵ and R⁶ are each independently a C₂₋₆ alkyl group which may have a substituent(s), or an amine in which two of R⁴, R⁵ and R⁶ are combined together to form a Cs alkylene chain whereby forming a 6-membered ring with the nitrogen atom to which they are bonded, and in this case, one of the alkylene chains may be replaced with O, more preferably an amine in which R⁴, R⁵ and R⁶ are each independently a C₂₋₆ alkyl group, and further preferably N,N-diisopropylethylamine or tri-n-propylamine.

An amount of the "organic tertiary amine having a specific structure" used in the present invention is preferably 0.01 equivalent to 50 equivalents based on the N-protected peptide represented by the formula (I), more preferably 0.1 equivalent to 20 equivalents, and further preferably 0.2 equivalent to 5 equivalents.

If the amount of the "organic tertiary amine having a specific structure" used in the present invention is less than 1 equivalent based on the N-protected peptide represented by the formula (I), the amount to be used in combination with the "other bases" is preferably 0.9 equivalent to 50 equivalents based on the N-protected peptide represented by the formula (I), more preferably 0.95 equivalent to 20 equivalents, and further preferably 1.0 equivalent to 5 equivalents.

An amount of the acid chloride to be used in the present invention is preferably 0.9 equivalent to 50 equivalents based on the N-protected peptide represented by the formula (I), more preferably 0.95 equivalent to 20 equivalents, and further preferably 1.0 equivalent to 5 equivalents.

The silylated amino acid or peptide to be used in the present invention is not particularly limited as long as it does not prohibit the reaction, and it can be prepared using, for example, an amino acid or a peptide and a silylating agent by the method described in Patent Documents 1 and 2.

The silylating agent to be used in the present invention is not particularly limited as long as it does not prohibit the reaction, and there may be mentioned, for example, N,O-bis(trimethylsilyl)acetamide, N,O-bis(trimethylsilyl)trifluoroacetamide, hexamethyldisilazane, N-methyl-N-trimethylsilylacetamide (MSA), N-methyl-N-trimethylsilyltrifluoroacetamide, N-(trimethylsilyl)acetamide, N-(trimethylsilyl)-diethylamine, N-(trimethylsilyl)dimethylamine, 1-(trimethylsilyl)imidazole, 3-(trimethylsilyl)-2-oxazolidone, trimethylsilyl cyanide, chlorotrimethylsilane, bromotrimethylsilane, iodotrimethylsilane and trimethylsilyltriflate, and preferably N, O-bis(trimethylsilyl)acetamide.

The solvent to be used in the present invention is not particularly limited as long as it does not prohibit the reaction, and examples thereof may be mentioned halogen-containing hydrocarbon solvents (for example, dichloromethane, chloroform), aromatic hydrocarbon solvents (for example, toluene, xylene), ether solvents (for example, tetrahydrofuran, 1,4-dioxane, cyclopentyl methyl ether, methyl-t-butyl ether), amide solvents (for example, N,N-dimethylformamide, N-methylpyrrolidone), nitrile solvents (for example, acetonitrile), etc. It is preferably nitrile solvents, amide solvents or ether solvents, and more preferably acetonitrile, tetrahydrofuran or N-methylpyrrolidone.

An amount of the solvent to be used in the present invention is preferably 100-fold by mass or less based on the N-protected peptide represented by the formula (I), more preferably 1-fold by mass to 50-fold by mass, and further preferably 5-fold by mass to 20-fold by mass.

The flow reactor to be used in the present invention is an apparatus in which a reagent can be fed continuously to a reactor and can continuously take out a reaction product, and usually uses a slender flow path having an inner diameter of from about 10 µm to 3 cm or so. In general, a device called to as a microreactor is a kind of a flow reactor.

In the present invention, a shape and a material of the mixer and a flow path are not particularly limited. For example, there may be used, as the mixer, a static type mixer such as a T-shaped tube, a static type mixer (trade name: Comet X-01) manufactured by Techno Applications, a static mixer (trade name: C type, T type) manufactured by Noritake Co., Limited, etc., a helix type mixer such as a helix type mixer (trade name: Spica) manufactured by YMC Co., Ltd., etc., and preferably a static type mixer (trade name: Comet X-01) manufactured by Techno Applications.

As a material of the mixer used in the present invention, stainless, glass and a polytetrafluoroethylene resin are preferable.

As the flow path used in the present invention, it is not particularly limited, preferably a tube having a cross-sectional area of from 80 µm² to 7 cm² and a length of from 1 cm to 300 m, and more preferably a tube having a cross-sectional area of from 0.2 mm² to 80 mm² and a length of from 0.1 m to 10 m.

As a material of the flow path used in the present invention, glass, stainless and a polytetrafluoroethylene resin are preferable.

A flow rate used in the present invention is not particularly limited, and is, for example, from 0.1 mL to 1 L per a minute, and preferably from 5 mL to 500 mL per a minute.

A reaction temperature in the present invention is not particularly limited, and it is possible from -40°C to a boiling point of the solvent to be used, and it is preferably carried out in the range of 0 to 40°C. In order to increase heat removal efficiency, the mixer portion may be set to a temperature lower than the subsequent portion.

The "peptide in which a C-terminal is elongated" obtained in the present invention is a compound represented by the formula (V): 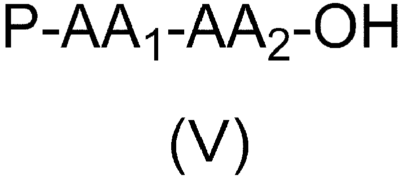 [wherein each symbol has the same meaning as defined above.].

In each reaction, when the reaction substrate has a hydroxy group, a mercapto group, an amino group, a carboxyl group or a carbonyl group (particularly when a functional group is possessed at the side chain of the amino acid or the peptide), a protective group which has generally been used in the peptide chemistry, etc., may be introduced into these groups, and a target compound can be obtained by removing the protective group after the reaction, if necessary.

Protection and deprotection can be carried out by subjecting to protection and deprotection reactions (for example, see Protective Groups in Organic Synthesis, Fourth edition, written by T. W. Greene, John Wiley & Sons Inc., 2006, etc.) using a generally known protective group(s).

### EXAMPLES

Hereinafter, the present invention will be explained in more detail by referring to Reference Synthetic Examples and Synthetic Examples, but the present invention is not limited to these Examples.

In the present specification, when the amino acid, etc., are indicated as abbreviations, each indication is based on abbreviation by IUPAC-IUB Commission on Biochemical Nomenclature or the conventional abbreviation in this field of the art.

The proton nuclear magnetic resonance (¹H-NMR) in Examples was measured by, unless otherwise specifically mentioned, JNM-ECP300 manufactured by JEOL, Ltd., or JNM-ECX300 manufactured by JEOL, Ltd., or Ascend^{™}500 manufactured by Bruker Co., in deuterated chloroform or deuterated dimethylsulfoxide solvent, and the chemical shift was shown by the δ value (ppm) when tetramethylsilane was used as the internal standard (0.0 ppm).

In the description of the NMR spectrum, "s" means singlet, "d" means doublet, "t" means triplet, "q" means quartet, "dd" means doublet of doublet, "dt" means doublet of triplet, "m" means multiplet, "br" means broad, "J" means coupling constant, "Hz" means hertz, and CDCl₃ means deuterated chloroform.

High performance liquid chromatography/mass analysis was measured by using, unless otherwise specifically mentioned, either of ACQUITY UPLC H-Class/QDa manufactured by Waters Corporation, ACQUITY UPLC H-Class/SQD2 manufactured by Waters Corporation or LC-20AD/Triple Tof5600 manufactured by Shimadzu Corporation.

In the description of the high performance liquid chromatography/mass analysis, ESI+ is a positive mode of the electrospray ionization method, M+H means a proton adduct and M+Na means a sodium adduct.

In the description of the high performance liquid chromatography/mass analysis, ESI- is a negative mode of the electrospray ionization method and M-H means a proton deficient.

For purification by silica gel column chromatography, unless otherwise specifically mentioned, either of Hi-Flash column manufactured by Yamazen Corporation, SNAP Ultra Silica Cartridge manufactured by Biotage, Silica gel 60 manufactured by Merck or PSQ60B manufactured by Fuji Silysia Chemical Ltd., was used.

Incidentally, the mixer used in the present Examples is, unless otherwise specified, a static type mixer (trade name: Comet X-01) manufactured by Techno Applications Co., Ltd.

### Reference Synthetic Example 1: Synthesis of Fmoc-Phe-Phe-OH

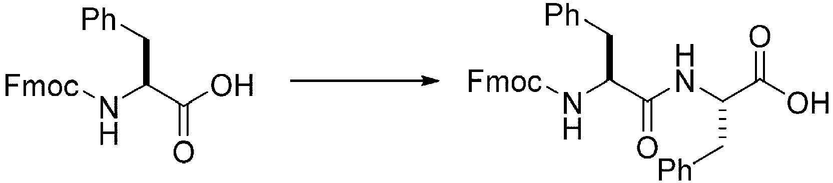

Phenylalanine (12.8 g, 78.5 mmol) was mixed with tetrahydrofuran (75.0 g), and after adding N,O-bistrimethylsilylacetamide (31.5 g, 157 mmol), the mixture was stirred at 40°C for 1 hour and then cooled to 0°C (silylated amino acid solution). Fmoc-Phe-OH (25.0 g, 64.5 mmol) and N-methylmorpholine (7.83 g, 78.5 mmol) were mixed with tetrahydrofuran (125 g), the mixture was cooled to 0°C, and after adding isobutyl chloroformate (9.70 g, 71.0 mmol) at once, the mixture was stirred for 2 minutes and the silylated amino acid solution was added thereto, and the resulting mixture was stirred for 1 hour. To the obtained reaction mixture was added water (50 g), and the mixture was concentrated under reduced pressure. The concentrated liquid was extracted with ethyl acetate (130 g) twice, and washing of the obtained organic layer with water was carried out three times. The obtained organic layer was concentrated under reduced pressure, acetonitrile (150 g) was added to the residue and the mixture was again concentrated under reduced pressure, acetonitrile (130 g) was added to the residue and the mixture was stirred for 0.5 hour, and then, the mixture was filtered to obtain white solid. To the obtained solid was added acetonitrile (150 g) and the mixture was stirred for 0.5 hour and after obtaining a solid by filtration, and the solid was subjected to drying under reduced pressure to obtain Fmoc-Phe-Phe-OH (26.7 g, Yield: 78%, diastereomeric excess: 99.7%de) as a white solid. MASS (ESI+) m/z; 535.34 (M+H)+

The diastereomeric excess was calculated by the analysis <Analytical condition A> using high performance liquid chromatography.

### <Analytical condition A>

High performance liquid chromatography: HPLC LC-20A manufactured by SHIMADZU Corporation
Column: Poroshell 120EC-C18 (2.7 µm, 3.0×100 mm) manufactured by Agilent Column oven temperature: 40°C
Eluent: Acetonitrile : 0.05 vol% phosphoric acid aqueous solution
45 : 55 (0 to 15 min), 45 : 55 to 95 : 5 (15 to 18 min), 95 : 5 (18 to 22 min) (v/v) Eluent rate: 0.8 mL/min
Detection wavelength: 210 nm

### Synthetic Example 1: Synthesis of Fmoc-Phe-Phe-Phe-OH using N-methylmorpholine

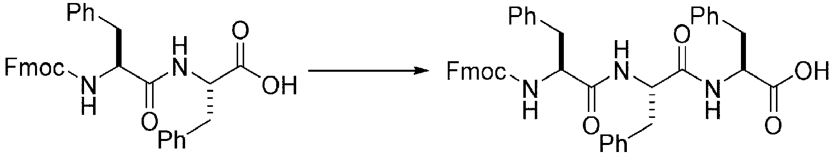

Fmoc-Phe-Phe-OH (2.00 g, 3.74 mmol) and N-methylmorpholine (0.45 g, 4.49 mmol) were mixed with tetrahydrofuran (8.89 g) and acetonitrile (7.86 g), and homogenized, which was made Solution 1. Isobutyl chloroformate (0.56 g, 4.11 mmol) was dissolved in acetonitrile (7.86 g), which was made Solution 2. Phenylalanine (0.74 g, 4.49 mmol) was mixed with acetonitrile (7.86 g), and after adding N,O-bistrimethyl-silylacetamide (1.83 g, 8.98 mmol) thereto, the mixture was stirred at 50°C for 1 hour and homogenized, and the temperature of the mixture was cooled to room temperature, which was made Solution 3. Solution 1 was fed at 12.1 mL per a minute and Solution 2 was fed at 5.80 mL per a minute, respectively, the solutions were mixed with a mixer and passed through a tube made of a polytetrafluoroethylene resin having an inner diameter of 1 mm and a length of 0.3 m, and Solution 3 was fed to the obtained mixed solution at 7.14 mL per a minute and the solutions were mixed with a mixer and passed through a tube made of a polytetrafluoroethylene resin having an inner diameter of 1 mm and a length of 1.0 m, and the obtained solution was collected in a container to which water (1.0 mL) had been added in advance for 1.5 minutes. The obtained solution was homogenized by adding N-methylpyrrolidone, and the quantitative yield of Fmoc-Phe-Phe-Phe-OH of the obtained solution was 97.6% and the diastereomeric excess was 99.8%de.

MASS (ESI+) m/z; 682.5 (M+H)+

The quantitative yield was calculated by a quantitative analysis method based on analysis <Analytical condition B> using high performance liquid chromatography.

### <Analytical condition B>

High performance liquid chromatography: HPLC LC-20A manufactured by SHIMADZU Corporation
Column: Poroshell 120EC-C18 (2.7 µm, 3.0×100 mm) manufactured by Agilent Column oven temperature: 40°C
Eluent: Acetonitrile : 0.05 vol% phosphoric acid aqueous solution
10 : 90 (0 min), 10 : 90 to 95 : 5 (0 to 11 min), 95 : 5 (11 to 15 min) (v/v)
Eluent rate: 0.7 mL/min
Detection wavelength: 210 nm

The diastereomeric excess was calculated by the analysis <Analytical condition C> using high performance liquid chromatography.

### <Analytical condition C>

High performance liquid chromatography: HPLC LC-20A manufactured by SHIMADZU Corporation
Column: Poroshell 120EC-C18 (2.7 µm, 3.0×100 mm) manufactured by Agilent Column oven temperature: 40°C
Eluent: Acetonitrile : 0.05 vol% phosphoric acid aqueous solution
50 : 50 (0 to 15 min), 50 : 50 to 95 : 5 (15 to 18 min), 95 : 5 (18 to 22 min) (v/v) Eluent rate: 0.7 mL/min
Detection wavelength: 210 nm

Quantitative analysis was carried out by the absolute calibration curve method using Fmoc-Phe-Phe-Phe-OH synthesized by the following procedure as a standard substance.

A part of the N-methylpyrrolidone solution obtained by the reaction was taken out, concentrated under reduced pressure, diluted with methylene chloride (10.0 g) and washed with water (5.0 g) twice. The obtained organic layer was concentrated under reduced pressure, acetonitrile (5.0 g) was added thereto, and the mixture was stirred and filtered to obtain a solid. Then, drying under reduced pressure was carried out to obtain Fmoc-Phe-Phe-Phe-OH (0.41 g) as a white solid.

The NMR of the standard substance is shown.

¹H NMR (300 MHz, CDCl₃):
δ 12.8 (1H, s), 8.35 (1H, d, J=8.1Hz), 8.06 (1H, d, J=7.5Hz), 7.87 (2H, d, J=7.8Hz), 7.52-7.62 (2H, m), 7.11-7.42 (20H, m), 4.42-7.62 (2H, m), 3.92-4.22 (4H, m), 2.62-3.10 (6H, m),

### Reference Synthetic Example 2: Synthesis of Fmoc-Phe-Phe-Phe-OH using N-ethylmorpholine

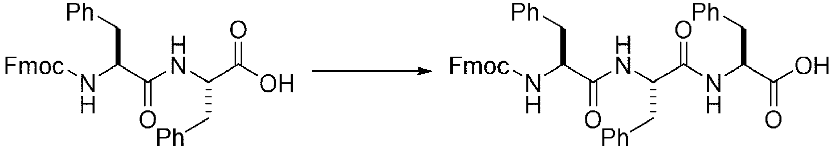

Fmoc-Phe-Phe-OH (0.50 g, 0.94 mmol) and N-ethylmorpholine (0.129 g, 1.13 mmol) were mixed with N-methylpyrrolidone (2.58 g) and chloroform (3.73 g), and homogenized, which was made Solution 1. Isobutyl chloroformate (0.153 g, 1.13 mmol) was dissolved in chloroform (3.73 g), which was made Solution 2. Phenylalanine (0.185 g, 1.13 mmol) was mixed with N-methylpyrrolidone (2.58 g), and after adding N,O-bistrimethylsilylacetamide (0.457 g, 2.26 mmol) thereto, the mixture was stirred at 50°C for 1 hour and homogenized, and the temperature of the mixture was cooled to room temperature, which was made Solution 3. Solution 1 was fed at 12.0 mL per a minute and Solution 2 was fed at 5.93 mL per a minute, respectively, the solutions were mixed with a mixer and passed through a tube made of a polytetrafluoroethylene resin having an inner diameter of 1 mm and a length of 0.3 m, and Solution 3 was fed to the obtained mixed solution at 7.02 mL per a minute and the solutions were mixed with a mixer and passed through a tube made of a polytetrafluoroethylene resin having an inner diameter of 1 mm and a length of 1.0 m, and the obtained solution was collected in a container to which water (0.5 mL) had been added in advance for 18 seconds. The obtained solution was homogenized by adding N-methylpyrrolidone, and the quantitative yield of Fmoc-Phe-Phe-Phe-OH of the obtained solution was 19.3%.

### Synthetic Example 2: Synthesis of Fmoc-Phe-Phe-Phe-OH using N-methylpiperidine

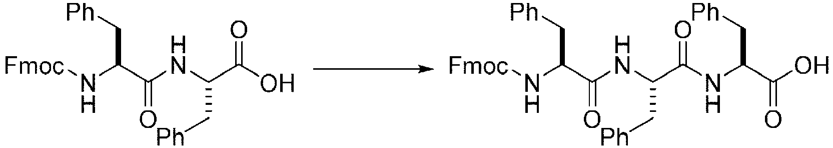

Fmoc-Phe-Phe-OH (0.50 g, 0.94 mmol) and N-methylpiperidine (0.112 g, 1.13 mmol) were mixed with N-methylpyrrolidone (2.58 g) and chloroform (3.73 g), and homogenized, which was made Solution 1. Isobutyl chloroformate (0.153 g, 1.13 mmol) was dissolved in chloroform (3.73 g), which was made Solution 2. Phenylalanine (0.185 g, 1.13 mmol) was mixed with N-methylpyrrolidone (2.58 g), and after adding N,O-bistrimethylsilylacetamide (0.457 g, 2.26 mmol) thereto, the mixture was stirred at 50°C for 1 hour and homogenized, and the temperature of the mixture was cooled to room temperature, which was made Solution 3. Solution 1 was fed at 12.0 mL per a minute and Solution 2 was fed at 5.93 mL per a minute, respectively, the solutions were mixed with a mixer and passed through a tube made of a polytetrafluoroethylene resin having an inner diameter of 1 mm and a length of 0.3 m, and Solution 3 was fed to the obtained mixed solution at 7.02 mL per a minute and the solutions were mixed with a mixer and passed through a tube made of a polytetrafluoroethylene resin having an inner diameter of 1 mm and a length of 1.0 m, and the obtained solution was collected in a container to which water (0.5 mL) had been added in advance for 18 seconds. The obtained solution was homogenized by adding N-methylpyrrolidone, and the quantitative yield of Fmoc-Phe-Phe-Phe-OH of the obtained solution was 93.0% and the diastereomeric excess was 99.6%de.

### Reference Synthetic Example 3: Synthesis of Fmoc-Phe-Phe-Phe-OH using N-ethylpiperidine

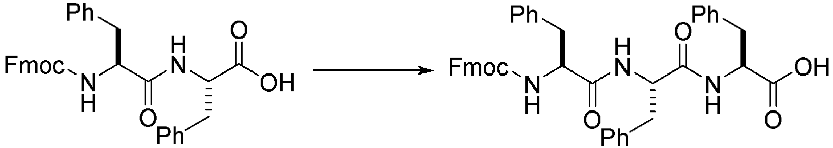

Fmoc-Phe-Phe-OH (0.50 g, 0.94 mmol) and N-ethylpiperidine (0.127 g, 1.13 mmol) were mixed with N-methylpyrrolidone (2.58 g) and chloroform (3.73 g), and homogenized, which was made Solution 1. Isobutyl chloroformate (0.153 g, 1.13 mmol) was dissolved in chloroform (3.73 g), which was made Solution 2. Phenylalanine (0.185 g, 1.13 mmol) was mixed with N-methylpyrrolidone (2.58 g), and after adding N,O-bistrimethylsilylacetamide (0.457 g, 2.26 mmol) thereto, the mixture was stirred at 50°C for 1 hour and homogenized, and the temperature of the mixture was cooled to room temperature, which was made Solution 3. Solution 1 was fed at 12.0 mL per a minute and Solution 2 was fed at 5.93 mL per a minute, respectively, the solutions were mixed with a mixer and passed through a tube made of a polytetrafluoroethylene resin having an inner diameter of 1 mm and a length of 0.3 m, and Solution 3 was fed to the obtained mixed solution at 7.02 mL per a minute and the solutions were mixed with a mixer and passed through a tube made of a polytetrafluoroethylene resin having an inner diameter of 1 mm and a length of 1.0 m, and the obtained solution was collected in a container to which water (0.5 mL) had been added in advance for 18 seconds. The obtained solution was homogenized by adding N-methylpyrrolidone, and the quantitative yield of Fmoc-Phe-Phe-Phe-OH of the obtained solution was 19.3%.

### Synthetic Example 3: Synthesis of Fmoc-Phe-Phe-Phe-OH using N-methyldiethylamine

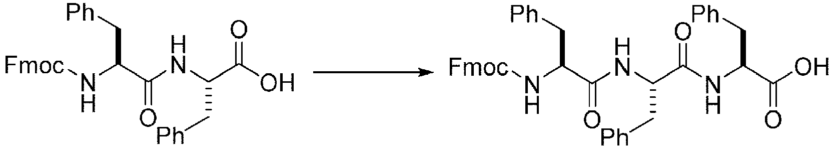

Fmoc-Phe-Phe-OH (0.50 g, 0.94 mmol) and N-methyldiethylamine (0.098 g, 1.13 mmol) were mixed with N-methylpyrrolidone (2.58 g) and chloroform (3.73 g), and homogenized, which was made Solution 1. Isobutyl chloroformate (0.153 g, 1.13 mmol) was dissolved in chloroform (3.73 g), which was made Solution 2. Phenylalanine (0.185 g, 1.13 mmol) was mixed with N-methylpyrrolidone (2.58 g), and after adding N,O-bistrimethylsilylacetamide (0.457 g, 2.26 mmol) thereto, the mixture was stirred at 50°C for 1 hour and homogenized, and the temperature of the mixture was cooled to room temperature, which was made Solution 3. Solution 1 was fed at 12.0 mL per a minute and Solution 2 was fed at 5.93 mL per a minute, respectively, the solutions were mixed with a mixer and passed through a tube made of a polytetrafluoroethylene resin having an inner diameter of 1 mm and a length of 0.3 m, and Solution 3 was fed to the obtained mixed solution at 7.02 mL per a minute and the solutions were mixed with a mixer and passed through a tube made of a polytetrafluoroethylene resin having an inner diameter of 1 mm and a length of 1.0 m, and the obtained solution was collected in a container to which water (0.5 mL) had been added in advance for 18 seconds. The obtained solution was homogenized by adding N-methylpyrrolidone, and the quantitative yield of Fmoc-Phe-Phe-Phe-OH of the obtained solution was 95.4% and the diastereomeric excess was 99.7%de.

### Reference Synthetic Example 4: Synthesis of Fmoc-Phe-Phe-Phe-OH using triethylamine

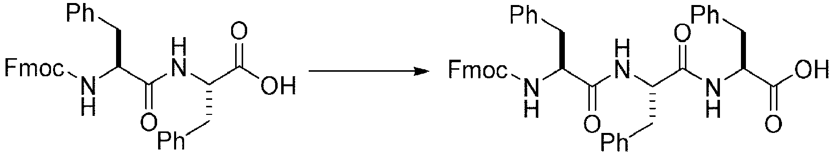

Fmoc-Phe-Phe-OH (0.50 g, 0.94 mmol) and triethylamine (0.114 g, 1.13 mmol) were mixed with N-methylpyrrolidone (2.58 g) and chloroform (3.73 g), and homogenized, which was made Solution 1. Isobutyl chloroformate (0.153 g, 1.13 mmol) was dissolved in chloroform (3.73 g), which was made Solution 2. Phenylalanine (0.185 g, 1.13 mmol) was mixed with N-methylpyrrolidone (2.58 g), and after adding N,O-bistrimethylsilylacetamide (0.457 g, 2.26 mmol) thereto, the mixture was stirred at 50°C for 1 hour and homogenized, and the temperature of the mixture was cooled to room temperature, which was made Solution 3. Solution 1 was fed at 12.0 mL per a minute and Solution 2 was fed at 5.93 mL per a minute, respectively, the solutions were mixed with a mixer and passed through a tube made of a polytetrafluoroethylene resin having an inner diameter of 1 mm and a length of 0.3 m, and Solution 3 was fed to the obtained mixed solution at 7.02 mL per a minute and the solutions were mixed with a mixer and passed through a tube made of a polytetrafluoroethylene resin having an inner diameter of 1 mm and a length of 1.0 m, and the obtained solution was collected in a container to which water (0.5 mL) had been added in advance for 18 seconds. The obtained solution was homogenized by adding N-methylpyrrolidone, and the quantitative yield of Fmoc-Phe-Phe-Phe-OH of the obtained solution was 33.2%.

### Synthetic Example 4: Synthesis of Fmoc-Phe-Phe-Phe-OH using N,N-dimethylbutylamine

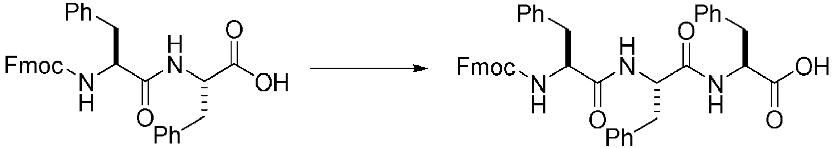

Fmoc-Phe-Phe-OH (0.50 g, 0.94 mmol) and N,N-dimethylbutylamine (0.114 g, 1.13 mmol) were mixed with N-methylpyrrolidone (2.58 g) and chloroform (3.73 g), and homogenized, which was made Solution 1. Isobutyl chloroformate (0.153 g, 1.13 mmol) was dissolved in chloroform (3.73 g), which was made Solution 2. Phenylalanine (0.185 g, 1.13 mmol) was mixed with N-methylpyrrolidone (2.58 g), and after adding N,O-bistrimethylsilylacetamide (0.457 g, 2.26 mmol) thereto, the mixture was stirred at 50°C for 1 hour and homogenized, and the temperature of the mixture was cooled to room temperature, which was made Solution 3. Solution 1 was fed at 12.0 mL per a minute and Solution 2 was fed at 5.93 mL per a minute, respectively, the solutions were mixed with a mixer and passed through a tube made of a polytetrafluoroethylene resin having an inner diameter of 1 mm and a length of 0.3 m, and Solution 3 was fed to the obtained mixed solution at 7.02 mL per a minute and the solutions were mixed with a mixer and passed through a tube made of a polytetrafluoroethylene resin having an inner diameter of 1 mm and a length of 1.0 m, and the obtained solution was collected in a container to which water (0.5 mL) had been added in advance for 18 seconds. The obtained solution was homogenized by adding N-methylpyrrolidone, and the quantitative yield of Fmoc-Phe-Phe-Phe-OH of the obtained solution was 95.4% and the diastereomeric excess was 99.8%de.

### Synthetic Example 5: Synthesis of Fmoc-Phe-Phe-Phe-OH using N,N-dimethylbenzylamine

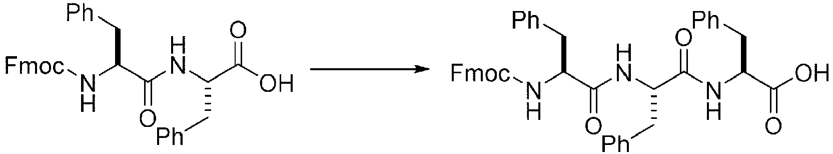

Fmoc-Phe-Phe-OH (0.50 g, 0.94 mmol) and N,N-dimethylbenzylamine (0.152 g, 1.13 mmol) were mixed with N-methylpyrrolidone (2.58 g) and chloroform (3.73 g), and homogenized, which was made Solution 1. Isobutyl chloroformate (0.153 g, 1.13 mmol) was dissolved in chloroform (3.73 g), which was made Solution 2. Phenylalanine (0.185 g, 1.13 mmol) was mixed with N-methylpyrrolidone (2.58 g), and after adding N,O-bistrimethylsilylacetamide (0.457 g, 2.26 mmol) thereto, the mixture was stirred at 50°C for 1 hour and homogenized, and the temperature of the mixture was cooled to room temperature, which was made Solution 3. Solution 1 was fed at 12.0 mL per a minute and Solution 2 was fed at 5.93 mL per a minute, respectively, the solutions were mixed with a mixer and passed through a tube made of a polytetrafluoroethylene resin having an inner diameter of 1 mm and a length of 0.3 m, and Solution 3 was fed to the obtained mixed solution at 7.02 mL per a minute and the solutions were mixed with a mixer and passed through a tube made of a polytetrafluoroethylene resin having an inner diameter of 1 mm and a length of 1.0 m, and the obtained solution was collected in a container to which water (0.5 mL) had been added in advance for 18 seconds. The obtained solution was homogenized by adding N-methylpyrrolidone, and the quantitative yield of Fmoc-Phe-Phe-Phe-OH of the obtained solution was 92.2% and the diastereomeric excess was 99.5%de.

### Synthetic Example 6: Synthesis of Fmoc-Phe-Phe-Phe-OH using N,N-diisopropylethylamine and N-methylmorpholine

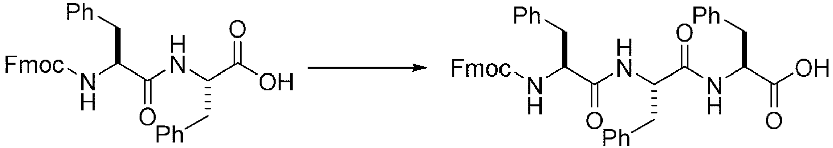

Fmoc-Phe-Phe-OH (0.50 g, 0.94 mmol), N,N-diisopropylethylamine (0.121 g, 0.94 mmol) and N-methylmorpholine (0.019 g, 0.19 mmol) were mixed with N-methylpyrrolidone (2.58 g) and chloroform (3.73 g), and homogenized, which was made Solution 1. Isobutyl chloroformate (0.153 g, 1.13 mmol) was dissolved in chloroform (3.73 g), which was made Solution 2. Phenylalanine (0.185 g, 1.13 mmol) was mixed with N-methylpyrrolidone (2.58 g), and after adding N,O-bistrimethylsilylacetamide (0.457 g, 2.26 mmol) thereto, the mixture was stirred at 50°C for 1 hour and homogenized, and the temperature of the mixture was cooled to room temperature, which was made Solution 3. Solution 1 was fed at 12.0 mL per a minute and Solution 2 was fed at 5.93 mL per a minute, respectively, the solutions were mixed with a mixer and passed through a tube made of a polytetrafluoroethylene resin having an inner diameter of 1 mm and a length of 0.3 m, and Solution 3 was fed to the obtained mixed solution at 7.02 mL per a minute and the solutions were mixed with a mixer and passed through a tube made of a polytetrafluoroethylene resin having an inner diameter of 1 mm and a length of 1.0 m, and the obtained solution was collected in a container to which water (0.5 mL) had been added in advance for 18 seconds. The obtained solution was homogenized by adding N-methylpyrrolidone, and the quantitative yield of Fmoc-Phe-Phe-Phe-OH of the obtained solution was 97.3% and the diastereomeric excess was 99.8%de.

### Reference Synthetic Example 5: Synthesis of Fmoc-Phe-Phe-Phe-OH using N,N-diisopropylethylamine

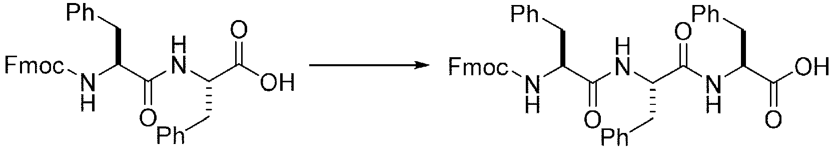

Fmoc-Phe-Phe-OH (0.50 g, 0.94 mmol) and N,N-diisopropylethylamine (0.145 g, 1.13 mmol) were mixed with N-methylpyrrolidone (2.58 g) and chloroform (3.73 g), and homogenized, which was made Solution 1. Isobutyl chloroformate (0.153 g, 1.13 mmol) was dissolved in chloroform (3.73 g), which was made Solution 2. Phenylalanine (0.185 g, 1.13 mmol) was mixed with N-methylpyrrolidone (2.58 g), and after adding N,O-bistrimethylsilylacetamide (0.457 g, 2.26 mmol) thereto, the mixture was stirred at 50°C for 1 hour and homogenized, and the temperature of the mixture was cooled to room temperature, which was made Solution 3. Solution 1 was fed at 12.0 mL per a minute and Solution 2 was fed at 5.93 mL per a minute, respectively, the solutions were mixed with a mixer and passed through a tube made of a polytetrafluoroethylene resin having an inner diameter of 1 mm and a length of 0.3 m, and Solution 3 was fed to the obtained mixed solution at 7.02 mL per a minute and the solutions were mixed with a mixer and passed through a tube made of a polytetrafluoroethylene resin having an inner diameter of 1 mm and a length of 1.0 m, and the obtained solution was collected in a container to which water (0.5 mL) had been added in advance for 18 seconds. The obtained solution was homogenized by adding N-methylpyrrolidone, and the quantitative yield of Fmoc-Phe-Phe-Phe-OH of the obtained solution was 2.0%.

### Reference Synthetic Example 6: Synthesis 1 of Fmoc-Phe-Phe-Phe-OH by batch system

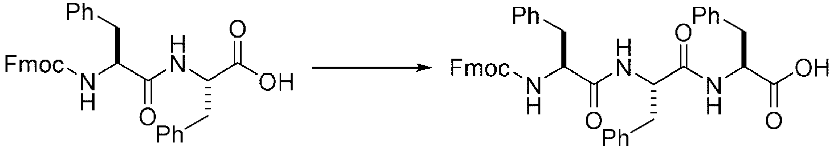

Phenylalanine (0.074 g, 0.44 mmol) was mixed with acetonitrile (1.58 g), and after adding N,O-bistrimethylsilylacetamide (0.183 g, 0.88 mmol) thereto, and the mixture was stirred at 50°C for 1 hour and cooled to 25°C (silylated amino acid solution). Fmoc-Phe-Phe-OH (0.20 g, 0.37 mmol) and N-methylmorpholine (0.045 g, 0.44 mmol) were mixed with tetrahydrofuran (0.89 g) and acetonitrile (0.79 g), the mixture was cooled to 0°C, and after adding isobutyl chloroformate (0.061 g, 0.44 mmol) at once, the resulting mixture was stirred for 30 minutes and the silylated amino acid solution was added thereto over 2 hours followed by stirring for 30 minutes. To the obtained reaction mixture were added water (1 g) and N-methylpyrrolidone and the mixture was homogenized. The quantitative yield of Fmoc-Phe-Phe-Phe-OH of the obtained solution was 42.0% and the diastereomeric excess was 95.6%de.

### Reference Synthetic Example 7: Synthesis 2 of Fmoc-Phe-Phe-Phe-OH by batch system

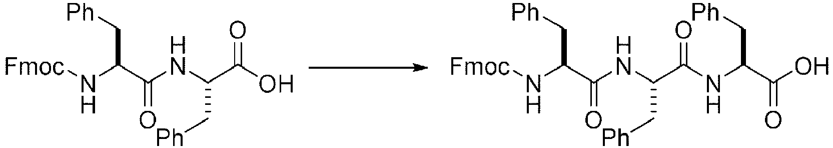

Phenylalanine (0.074 g, 0.44 mmol) was mixed with acetonitrile (1.58 g), and after adding N,O-bistrimethylsilylacetamide (0.183 g, 0.88 mmol) thereto, and the mixture was stirred at 50°C for 1 hour and cooled to 25°C (silylated amino acid solution). Fmoc-Phe-Phe-OH (0.20 g, 0.37 mmol) and N-methylmorpholine (0.045 g, 0.44 mmol) were mixed with tetrahydrofuran (0.89 g) and acetonitrile (0.79 g), and after adding isobutyl chloroformate (0.061 g, 0.44 mmol) at 25°C at once, the resulting mixture was stirred for 30 minutes and the silylated amino acid solution was added thereto over 2 hours followed by stirring for 30 minutes. To the obtained reaction mixture were added water (1 g) and N-methylpyrrolidone and the mixture was homogenized, and the quantitative yield of Fmoc-Phe-Phe-Phe-OH of the thus obtained solution was 21.7% and the diastereomeric excess was 30.1%de.

### Synthetic Example 7: Synthesis of Fmoc-Phe-Phe-Cys(Trt)-OH

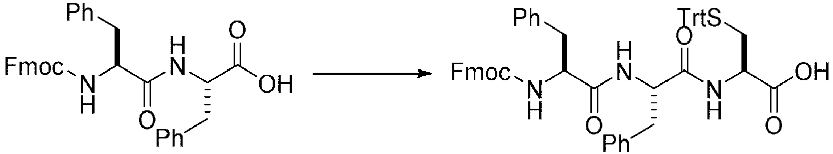

Fmoc-Phe-Phe-OH (2.00 g, 3.74 mmol) and N-methylmorpholine (0.454 g, 4.49 mmol) were mixed with tetrahydrofuran (8.89 g) and acetonitrile (7.86 g), and homogenized, which was made Solution 1. Isobutyl chloroformate (0.562 g, 4.11 mmol) was dissolved in acetonitrile (7.863 g), which was made Solution 2. S-tritylcysteine (1.632 g, 4.49 mmol) was mixed with acetonitrile (17.3 g), and after adding N,O-bistrimethyl-silylacetamide (1.827 g, 8.98 mmol) thereto, the mixture was stirred at 50°C for 1 hour and homogenized, and the temperature of the mixture was cooled to room temperature, which was made Solution 3. Solution 1 was fed at 9.4 mL per a minute and Solution 2 was fed at 4.5 mL per a minute, respectively, the solutions were mixed with a mixer and passed through a tube made of a polytetrafluoroethylene resin having an inner diameter of 1 mm and a length of 0.3 m, and Solution 3 was fed to the obtained mixed solution at 11.1 mL per a minute and the solutions were mixed with a mixer and passed through a tube made of a polytetrafluoroethylene resin having an inner diameter of 1 mm and a length of 1.0 m, and the obtained solution was collected in a container to which an aqueous 10% potassium carbonate solution (10 mL) had been added in advance for 125 seconds. The obtained solutions were separated, an aqueous 10% potassium carbonate solution (15 g) was added thereto again and the liquids were separated, and the organic layer was washed with an aqueous 10% sodium chloride solution . To the obtained organic layer were added an aqueous 10% sodium chloride solution (5 g) and an aqueous 10% ammonium chloride solution (5 g), and the liquids were separated twice. The obtained organic layer was concentrated and purified by silica gel chromatography to obtain Fmoc-Phe-Phe-Cys(Trt)-OH (2.74 g, Yield: 94%, diastereomeric excess: 99.2%de) as a white solid.

MASS (ESI+) m/z; 902.36 (M+Na)+

The diastereomeric excess was calculated by the analysis <Analytical condition D> using high performance liquid chromatography.

### <Analytical condition D>

High performance liquid chromatography: HPLC LC-20A manufactured by SHIMADZU Corporation
Column: Poroshell 120EC-C18(2.7µm, 3.0×100mm) manufactured by Agilent Column oven temperature: 40°C
Eluent: Acetonitrile : 0.05 vol% phosphoric acid aqueous solution
58 : 42 (0-27 min), 58 : 42-95: 5 (27-32 min), 95 : 5 (32-34 min) (v/v)
Eluent rate: 0.9 mL/min
Detection wavelength: 210 nm

### Synthetic Example 8: Synthesis of Fmoc-Phe-Phe-Cys(Trt)-Tyr(t-Bu)-OH

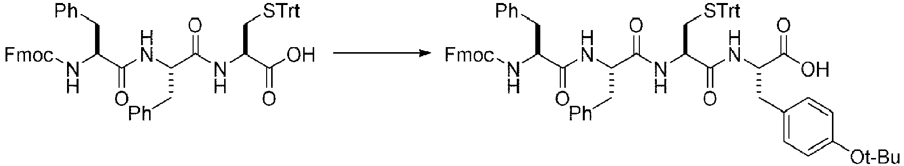

Fmoc-Phe-Phe-Cys(Trt)-OH (2.00 g, 2.27 mmol) and N-methylmorpholine (0.276 g, 2.72 mmol) were mixed with tetrahydrofuran (8.89 g) and acetonitrile (7.86 g), and homogenized, which was made Solution 1. Isobutyl chloroformate (0.341 g, 2.50 mmol) was dissolved in acetonitrile (7.863 g), which was made Solution 2. O-t-Bu-tyrosine (0.647 g, 2.72 mmol) was mixed with acetonitrile (7.86 g), and after adding N,O-bistrimethylsilylacetamide (1.110 g, 5.45 mmol) thereto, the mixture was stirred at 50°C for 1 hour and homogenized, and the temperature of the mixture was cooled to room temperature, which was made Solution 3. Solution 1 was fed at 12.4 mL per a minute and Solution 2 was fed at 5.8 mL per a minute, respectively, the solutions were mixed with a mixer and passed through a tube made of a polytetrafluoroethylene resin having an inner diameter of 1 mm and a length of 0.3 m, and Solution 3 was fed to the obtained mixed solution at 6.8 mL per a minute and the solutions were mixed with a mixer and passed through a tube made of a polytetrafluoroethylene resin having an inner diameter of 1 mm and a length of 1.0 m, and the obtained solution was collected in a container to which an aqueous 10% potassium carbonate solution (10 mL) had been added in advance for 90 seconds. The obtained solutions were separated, an aqueous 10% potassium carbonate solution (15 g) was added thereto again and the liquids were separated, and the organic layer was washed with an aqueous 10% sodium chloride solution. The obtained organic layer was concentrated and purified by silica gel chromatography, and after concentrating the collected solution, hexane (40 g) was mixed therewith and the precipitated solid was filtered and dried to obtain Fmoc-Phe-Phe-Cys(Trt)-Tyr(t-Bu)-OH (1.95 g, Yield: 92%, diastereomeric excess: 99.6%de) as a white solid.

MASS (ESI+) m/z; 1121.64 (M+Na)+

The diastereomeric excess was calculated by the analysis <Analytical condition E> using high performance liquid chromatography.

### <Analytical condition E>

High performance liquid chromatography: HPLC LC-20A manufactured by SHIMADZU Corporation
Column: Poroshell 120EC-C18 (2.7µm, 3.0×100mm) manufactured by Agilent Column oven temperature: 40°C
Eluent: Acetonitrile : 0.05 vol% phosphoric acid aqueous solution
70 : 430 (0 to 27 min), 70 : 30 to 95 : 5 (27 to 32 min), 95 : 5 (32 to 34 min) (v/v) Eluent rate: 0.9 mL/min
Detection wavelength: 210 nm

### Reference Synthetic Example 8: Synthesis of Fmoc-Phe-D-Phg-OH

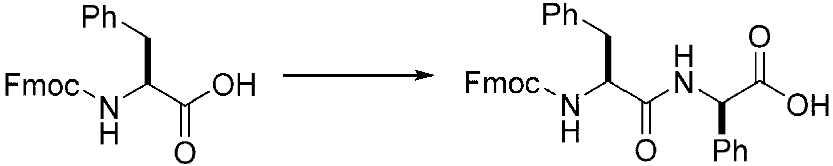

Fmoc-Phe-OH (2.00 g, 5.16 mmol) and N-methylmorpholine (0.627 g, 6.19 mmol) were mixed with tetrahydrofuran (8.89 g) and acetonitrile (7.86 g), and homogenized, which was made Solution 1. Isobutyl chloroformate (0.776 g, 5.68 mmol) was dissolved in acetonitrile (7.863 g), which was made Solution 2. D-phenylglycine (0.936 g, 6.19 mmol) was mixed with acetonitrile (7.86 g), and after adding N,O-bistrimethylsilylacetamide (3.255 g, 12.4 mmol) thereto, the mixture was stirred at 50°C for 1 hour and homogenized, and the temperature of the mixture was cooled to room temperature, which was made Solution 3. Solution 1 was fed at 11.5 mL per a minute and Solution 2 was fed at 5.6 mL per a minute, respectively, the solutions were mixed with a mixer and passed through a tube made of a polytetrafluoroethylene resin having an inner diameter of 1 mm and a length of 0.3 m, and Solution 3 was fed to the obtained mixed solution at 7.8 mL per a minute and the solutions were mixed with a mixer and passed through a tube made of a polytetrafluoroethylene resin having an inner diameter of 1 mm and a length of 1.0 m, and the obtained solution was collected for 105 seconds in a container to which water (2 mL) had been added in advance. With the obtained solution was mixed acetonitrile (80 g), and the precipitated solid was filtered and dried to obtain Fmoc-Phe-D-Phg-OH (1.50 g, Yield: 62%) as a white solid.

MASS (ESI+) m/z; 521.3 (M+Na)+

### Synthetic Example 9: Synthesis of Fmoc-Phe-D-Phg-Phe-OH

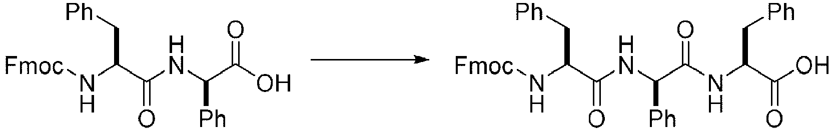

Fmoc-Phe-D-Phg-OH (0.50 g, 0.96 mmol) and N-methylmorpholine (0.117 g, 1.15 mmol) were mixed with N-methylpyrrolidone (2.58 g) and acetonitrile (1.97 g), and homogenized, which was made Solution 1. Isobutyl chloroformate (0.144 g, 1.06 mmol) was dissolved in acetonitrile (1.97 g), which was made Solution 2. Phenylalanine (0.19 g, 1.06 mmol) was mixed with acetonitrile (1.97 g), and after adding N,O-bistrimethylsilylacetamide (0.469 g, 2.30 mmol) thereto, the mixture was stirred at 50°C for 1 hour and homogenized, and the temperature of the mixture was cooled to room temperature, which was made Solution 3. Solution 1 was fed at 11.9 mL per a minute and Solution 2 was fed at 5.8 mL per a minute, respectively, the solutions were mixed with a mixer and passed through a tube made of a polytetrafluoroethylene resin having an inner diameter of 1 mm and a length of 0.3 m, and Solution 3 was further fed at 7.2 mL per a minute to the obtained mixed solution and the resulting mixture was mixed with a mixer and passed through a tube made of a polytetrafluoroethylene resin having an inner diameter of 1 mm and a length of 1.0 m, and the obtained solution was collected for 20 seconds in a container to which water (1 mL) had been added in advance. The obtained solution was concentrated under reduced pressure and acetonitrile (25 g) was mixed therewith, and the precipitated solid was filtered and dried to obtain Fmoc-Phe-D-Phg-Phe-OH (0.45 g, Yield: 95%) as a white solid.

MASS (ESI+) m/z; 668.4 (M+Na)+

The diastereomeric excess was calculated by the analysis <Analytical condition C> using high performance liquid chromatography.

### Synthetic Example 10: Synthesis of Cbz-Phe-Phe-Leu-OH

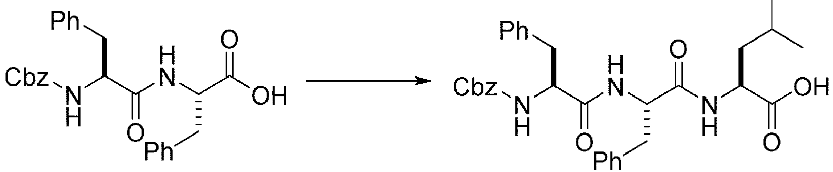

Cbz-Phe-Phe-OH (2.00 g, 4.48 mmol) and N-methylmorpholine (0.591 g, 5.38 mmol) were mixed with tetrahydrofuran (8.89 g) and acetonitrile (7.86 g), and homogenized, which was made Solution 1. Isobutyl chloroformate (0.641 g, 4.93 mmol) was dissolved in acetonitrile (7.863 g), which was made Solution 2. Leucine (0.705 g, 5.38 mmol) was mixed with acetonitrile (7.86 g), and after adding N,O-bistrimethyl-silylacetamide (2.187 g, 10.8 mmol) thereto, the mixture was stirred at 50°C for 1 hour and homogenized, and the temperature of the mixture was cooled to room temperature, which was made Solution 3. Solution 1 was fed at 12.0 mL per a minute and Solution 2 was fed at 5.8 mL per a minute, respectively, the solutions were mixed with a mixer and passed through a tube made of a polytetrafluoroethylene resin having an inner diameter of 1 mm and a length of 0.3 m, and Solution 3 was further fed at 73 mL per a minute to the obtained mixed solution and the resulting mixture was mixed with a mixer and passed through a tube made of a polytetrafluoroethylene resin having an inner diameter of 1 mm and a length of 1.0 m, and the obtained solution was collected for 100 seconds in a container to which 5 wt% hydrochloric acid (5 g) had been added in advance. The obtained solution was concentrated under reduced pressure, ethyl acetate (30 g) was added to the residue and the liquids were separated, and the organic layer was washed with an aqueous 10 wt% sodium chloride solution (5 g) twice. The obtained organic layer was concentrated and hexane (40 g) was added thereto and the resulting mixture was stirred, and the precipitated solid was filtered and dried to obtain Cbz-Phe-Phe-Leu-OH (2.17 g, Yield: 96%, diastereomeric excess: 100%de) as a white solid.

MASS (ESI+) m/z; 560.4 (M+H)+

The diastereomeric excess was calculated by the analysis <Analytical condition A> using high performance liquid chromatography.

### Reference Synthetic Example 9: Synthesis of H-Phe-Phe-Leu-OH

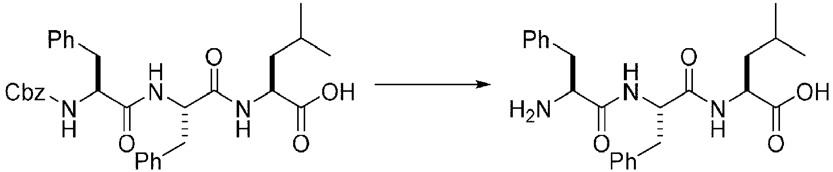

Cbz-Phe-Phe-Leu-OH (1.50 g, 2.68 mmol) was added to tetrahydrofuran (10 g) and methanol (10 g) and dissolved therein, and a 10 wt% palladium carbon (0.15 g, available from NE Chemcat Corporation, PE Type) was added to the solution and the resulting mixture was stirred at 21°C by hydrogen substitution for 1 hour. The obtained solution was filtered, N,N-dimethylformamide (150 g) was added to the filtrate and the mixture was filtered to obtain Filtrate 1. To the obtained filtrate was again added N,N-dimethylformamide (150 g) and after stirring the mixture, it was filtered to obtain Filtrate 2. Filtrate 1 and Filtrate 2 were mixed and concentrated under reduced pressure, and acetonitrile (20 g) was added and after the mixture was stirred, filtration was carried out and the obtained solid was dried to obtain H-Phe-Phe-Leu-OH (1.03 g, Yield: 90%) as a gray solid.

MASS (ESI+) m/z; 426.4 (M+H)+

### Synthetic Example 11: Synthesis of Fmoc-Phe-Phe-Cys(Trt)-Tyr(t-Bu)-Phe-Phe-Leu-OH

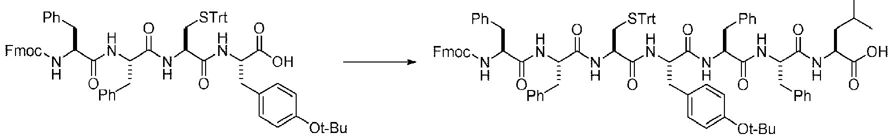

Fmoc-Phe-Phe-Cys(Trt)-Tyr(t-Bu)-OH (0.55 g, 0.50 mmol) and N-methylmorpholine (0.066 g, 0.60 mmol) were mixed with tetrahydrofuran (2.45 g) and acetonitrile (2.16 g), and homogenized, which was made Solution 1. Isobutyl chloroformate (0.075 g, 0.55 mmol) was dissolved in acetonitrile (2.16 g), which was made Solution 2. H-Phe-Phe-Leu-OH (0.255 g, 0.60 mmol) was mixed with acetonitrile (2.16 g), and after adding N,O-bistrimethylsilylacetamide (0.244 g, 1.20 mmol) thereto, the mixture was
stirred at room temperature and homogenized, which was made Solution 3. Solution 1 was fed at 12.3 mL per a minute and Solution 2 was fed at 5.8 mL per a minute, respectively, the solutions were mixed with a mixer and passed through a tube made of a polytetrafluoroethylene resin having an inner diameter of 1 mm and a length of 0.3 m,
and Solution 3 was further fed at 6.9 mL per a minute to the obtained mixed solution and the resulting mixture was mixed with a mixer and passed through a tube made of a polytetrafluoroethylene resin having an inner diameter of 1 mm and a length of 1.0 m,
and the obtained solution was collected for 20 seconds in a container to which water (1 g) had been added in advance. With the obtained solution was mixed water (4 g) and the mixture was stirred, and the precipitated solid was filtered and dried to obtain Fmoc-Phe-Phe-Cys(Trt)-Tyr(t-Bu)-Phe-Phe-Leu-OH (0.43 g, Yield: 84%, diastereomeric excess: 99.4%de) as a white solid.

MASS (ESI+) m/z; 1507(M+H)+

The diastereomeric excess was calculated by the analysis <Analytical condition F> using high performance liquid chromatography.

### <Analytical condition F>

High performance liquid chromatography: HPLC LC-20A manufactured by SHIMADZU Corporation
Column: Poroshell 120EC-C18 (2.7 µm, 3.0×100 mm) manufactured by Agilent Column oven temperature: 40°C
Eluent: Acetonitrile : 0.05 vol% phosphoric acid aqueous solution
80 : 20 (0 to 27 min), 80 : 20 to 95 : 5 (27 to 32 min), 95 : 5 (32 to 34 min) (v/v) Eluent rate: 0.9 mL/min
Detection wavelength: 210 nm

### Synthetic Example 12: Synthesis of Fmoc-Phe-Phe-MeGly-OH

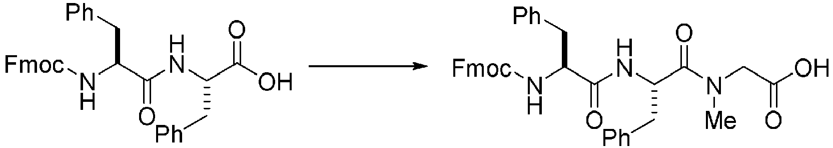

Fmoc-Phe-Phe-OH (2.00 g, 3.74 mmol) and N-methylmorpholine (0.454 g, 4.49 mmol) were mixed with tetrahydrofuran (8.89 g) and acetonitrile (7.86 g), and homogenized, which was made Solution 1. Isobutyl chloroformate (0.562 g, 4.11 mmol) was dissolved in acetonitrile (7.863 g), which was made Solution 2. Sarcosine (0.667 g, 7.48 mmol) was mixed with acetonitrile (7.86 g), and after adding N,O-bistrimethyl-silylacetamide (3.04 g, 15.0 mmol) thereto, the mixture was stirred at 50°C for 1 hour and homogenized, and the temperature of the mixture was cooled to room temperature, which was made Solution 3. Solution 1 was fed at 11.7 mL per a minute and Solution 2 was fed at 5.6 mL per a minute, respectively, the solutions were mixed with a mixer and passed through a tube made of a polytetrafluoroethylene resin having an inner diameter of 1 mm and a length of 0.3 m, and Solution 3 was further fed at 7.7 mL per a minute to the obtained mixed solution and the resulting mixture was mixed with a mixer and passed through a tube made of a polytetrafluoroethylene resin having an inner diameter of 1 mm and a length of 1.0 m, and the obtained solution was collected for 107 seconds in a container to which 5 wt% hydrochloric acid (10 g) had been added in advance. The obtained solution was concentrated under reduced pressure, ethyl acetate (30 g) was added thereto and the liquids were separated, and the organic layer was washed with an aqueous 10 wt% sodium chloride solution (10 g) twice. The obtained organic layer was concentrated and purified by silica gel chromatography to obtain Fmoc-Phe-Phe-MeGly-OH (2.06 g, Yield: 96%, diastereomeric excess: 99.7%de) as a white solid.

MASS (ESI+) m/z; 606.4 (M+H)+

The diastereomeric excess was calculated by the analysis <Analytical condition A> using high performance liquid chromatography.

### UTILIZABILITY IN INDUSTRY

According to the present invention, a method for producing a peptide with high efficiency can be provided.

## Claims

1. A method for producing a peptide which comprises the following step (1):
(1) a step of reacting a peptide in which a C-terminal carboxy group is activated which is obtained by reacting an N-protected peptide represented by the formula (I): 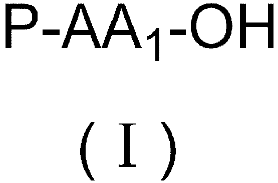
wherein AA₁ represents a group derived from a peptide comprising a 2 to 20 amino acids, and P represents an N-terminal protective group,
a tertiary amine represented by the formula (II): 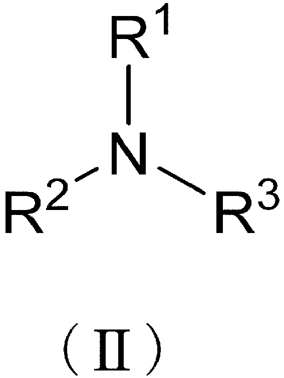
wherein among R¹, R² and R³, one or two is/are a methyl group(s), the remainder(s) is/are an aliphatic hydrocarbon group(s) which may have a substituent(s), when one of R¹, R² and R³ is a methyl group, the remaining two may form a 6 to 7-membered ring, together with the nitrogen atom to which they are bonded, by combining them to form a C₅₋₆ alkylene chain and, in this case, one of the alkylene chains may be substituted with O or NR⁸ where R⁸ represents an aliphatic hydrocarbon group which may have a substituent(s),
and an acid halide in a flow reactor,
with a silylated amino acid or peptide obtained by reacting an amino acid or a peptide represented by the formula (III): 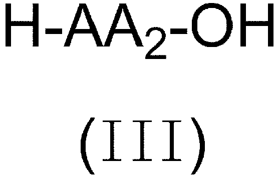
wherein AA₂ represents an amino acid or a group derived from a peptide having 2 to 20 residues,
with a silylating agent,
in a flow reactor.

2. The method for producing a peptide according to Claim 1, wherein an amount of the tertiary amine represented by the formula (II) to be used is 0.05 mol equivalent to 1 mol equivalent based on the N-protected peptide represented by the formula (I), and further using a tertiary amine represented by the formula (IV) 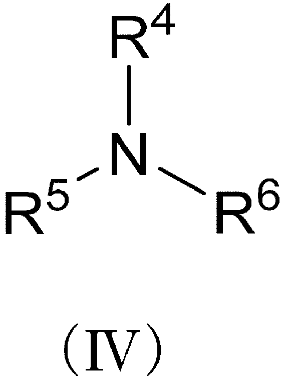 wherein R⁴, R⁵ and R⁶ each independently represent an aliphatic hydrocarbon group, provided that a methyl group is excluded, which may have a substituent(s), two of R⁴, R⁵ and R⁶ may form a 6 to 7-membered ring, together with the nitrogen atom to which they are bonded, by combining them to form a C₅₋₆ alkylene chain and, in this case, one of the alkylene chains may be replaced with O or NR⁸ where R⁸ represents an aliphatic hydrocarbon group which may have a substituent(s).

3. The method for producing a peptide according to Claim 2, wherein a combined amount of the tertiary amine represented by the formula (IV) and the tertiary amine represented by the formula (II) to be used is 1.0 to 10 mol equivalents based on the N-protected peptide represented by the formula (I).

4. The method for producing a peptide according to any one of Claims 1 to 3, wherein the acid halide is an alkyl chloroformate, carboxylic acid chloride, sulfonyl chloride or phosphoryl chloride.

5. The method for producing a peptide according to any one of Claims 1 to 4, wherein the acid halide is a C₁₋₆ alkyl chloroformate.

6. The method for producing a peptide according to any one of Claims 1 to 5, wherein the acid halide is isobutyl chloroformate.

7. The method for producing a peptide according to any one of Claims 1 to 6, wherein the tertiary amine represented by the formula (II) is N,N-dimethylbutylamine, N,N-dimethylbenzylamine, N-methyldiethylamine, N-methylpiperidine or N-methylmorpholine.

8. The method for producing a peptide according to any one of Claims 2 to 7, wherein the tertiary amine represented by the formula (IV) is N,N-diisopropylethylamine or tri-n-propylamine.

9. The method for producing a peptide according to any one of Claims 1 to 8, wherein the amino acid or the peptide is constituted by an α-amino acid.

10. The method for producing a peptide according to any one of Claims 1 to 9, wherein the protective group of the N-terminal of the N-protected peptide is a carbamate-based protective group.

11. The method for producing a peptide according to any one of Claims 1 to 10, wherein the protective group of the N-terminal of the N-protected peptide is a benzyloxycarbonyl group, a 9-fluorenylmethoxycarbonyl group or a t-butoxycarbonyl group.

12. The method for producing a peptide according to any one of Claims 1 to 11, wherein the silylating agent is N,O-bis(trimethylsilyl)acetamide.
